# EUROPEAN PATENT APPLICATION

(11) **EP 4 195 217 A1**
(43) Date of publication of application: **14.06.2023**
(21) Application number: 22161375.5
(22) Date of filing: 10.03.2022
(51) Int. Cl.: G16H 40/20, G16H 30/20, G16H 30/40

(54) **SUPPORT TOOLS FOR RADIOLOGIST IMAGE REVIEW FOR IMMEDIATE FEEDBACK**

(30) Priority: 13.12.2021 US 202163288688 P
(71) Applicant: Koninklijke Philips N.V., 5656 AG Eindhoven (NL)
(72) Inventor: STAROBINETS, Olga, Eindhoven (NL); DALAL, Sandeep, Madhukar, Eindhoven (NL); KOKER, Ekin, Eindhoven (NL); LUI, Saifeng, Eindhoven (NL)
(74) Representative: Philips Intellectual Property & Standards

(57) **Abstract**

A non-transitory computer readable medium (26s) stores instructions executable by at least one electronic processor (14s) to perform a method (100) to coordinate radiologist review of a medical imaging procedure performed using a medical imaging device (2). The method includes acquiring a video (17) of the medical imaging device; determining a review time for the medical imaging procedure; providing a notification to a remote electronic processing device (12) operable by a radiologist of the determined review time; and at the review time, extracting at least one review image (38) from the video and making the at least one review image available at the remote electronic processing device.

## Description

### FIELD OF THE INVENTION

The following relates generally to the remote imaging assistance arts, remote imaging examination monitoring arts, radiology imaging reading arts, and related arts.

### BACKGROUND OF THE INVENTION

During a typical image acquisition procedure, the acquired images are reviewed by a radiologist. This review can take place while the patient is still in the scanner and additional images can be acquired if needed. If a radiologist finds presented images satisfactory, the examination is ended, and the patient is released. Alternatively, if the radiologist concludes the presented images are in some way deficient, then additional images are acquired. Reasons for requiring additional images may include, by way of nonlimiting illustrative example, incorrect field-of-view (FOV), failure to acquire images from all required views, identification of an incidental finding that should be further imaged, and so forth. This approach of having a radiologist review the images before releasing the patient ensures good diagnostic quality imaging, a complete set of images, eliminates the need for call-backs, and provides opportunity for a better assessment of potential incidental findings.

Radiology scans of suboptimal quality may nonetheless be read, and can carry compromised diagnostic value or result in patient return for repeat scanning. Radiology call backs are uncommon, but are largely preventable and add an unnecessary burden on both the patient and the hospital. A traditional radiology workflow in most imaging centers involves image acquisition by a technologist with a radiologist reading images and summarizing findings in a radiology report at a later time (ranging from minutes to days after exam completion).

The following discloses certain improvements to overcome these problems and others.

### SUMMARY OF THE INVENTION

In one aspect, a non-transitory computer readable medium stores instructions executable by at least one electronic processor to perform a method to coordinate radiologist review of a medical imaging procedure performed using a medical imaging device. The method includes acquiring a video of the medical imaging device; determining a review time for the medical imaging procedure; providing a notification to a remote electronic processing device operable by a radiologist of the determined review time; and at the review time, extracting at least one review image from the video and making the at least one review image available at the remote electronic processing device.

In another aspect, a non-transitory computer readable medium stores instructions executable by at least one electronic processor to perform a method to coordinate radiologist review of a medical imaging procedure performed using a medical imaging device. The method includes acquiring a video of the medical imaging device; determining a review time for the medical imaging procedure; at the review time, extracting at least one review image from the video and making the at least one review image available at the remote electronic processing device; and transmitting the at least one review image to the remote electronic processing device. The medical imaging procedure acquires at least one clinical image corresponding to the at least one review image.

In another aspect, a method to coordinate review of a medical procedure performed using a medical device includes acquiring a video of the medical device; determining a review time for the medical procedure; providing a notification to a remote electronic processing device operable by a medical professional of the determined review time; and at the review time, extracting at least one review image from the video and making the at least one review image available at the remote electronic processing device.

One advantage resides in enhanced timeliness in radiologist review of images before an imaging examination procedure is completed.

Another advantage resides in screen-scraping a console screen of a medical imaging device controller to review acquired images of a patient before an imaging examination procedure is completed.

Another advantage resides in acquiring images from a camera in a medical imaging device bay to review acquired images of a patient before an imaging examination procedure is completed.

Another advantage resides in reducing a number of procedures of re-acquiring images of a patient.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 diagrammatically shows an illustrative apparatus for coordinating radiologist review of a medical imaging procedure in accordance with the present disclosure.
Fig. 2 shows an example flow chart of operations suitably performed by the apparatus of Fig. 1 to provide a local operator with the assistance of a remote expert who is an expert imaging technician during a medical imaging examination.
Fig. 3 shows an example flow chart of operations suitably performed by the apparatus of Fig. 1 to leverage the system of Fig. 1 to provide timely notification to a remote expert who is an on-call radiologist that an imaging examination is complete or is near completion, and that images are or will soon be available for review by the radiologist prior to releasing the patient from the imaging examination.

### DETAILED DESCRIPTION OF EMBODIMENTS

The following relates to a system with tools for expediting radiologist review of clinical images prior to unloading the patient from an imaging scanner.

In some radiology laboratories, especially in Europe, it is present practice to have a radiologist on call to review radiology images prior to unloading the patient. This advantageously ensures the images are suitable for the intended diagnostic task, as further images can be acquired while the patient is still in the scanner rather than requiring a call-back to repeat the entire imaging session at a later date.

However, the radiologist is usually notified that images are available for review by telephone or other active call from the imaging technician. As the technician is busy performing the image acquisition, this call to the radiologist may not occur until the imaging is completed, introducing a potentially substantial delay as the radiologist is then called. Furthermore, the radiologist typically receives the high resolution clinical images for review by way of a Picture Archiving and Communication System (PACS), and the process of uploading the high resolution clinical images to the PACS and then downloading them to the radiologist can be lengthy.

In some embodiments disclosed herein, information extracted from the controller display can be used to estimate the time remaining in the imaging examination, and the disclosed system can then automatically notify the radiologist in a "just-in-time" fashion so that the radiologist is aware the images will be available ahead of time. Various approaches can be used for estimating end-of-examination, such as detecting contrast agent injection (usually done as the last stage of an imaging examination since the contrast agent would interfere with other stages) or directly reading scan "time remaining" text shown on the display controller via optical character recognition (OCR).

Additionally, the screen scrape of the controller display can be mined to extract images for the radiologist to review. This approach is based in part on the recognition herein that the radiologist review of the images immediately after the scan is not a diagnostic review or a clinical reading of the radiology examination. Rather, it is intended to detect significant errors that could adversely impact the diagnostic quality of the images. Lower resolution images from the scraped screen are sufficient to detect problems such as an incorrect field of view, incorrect and/or incomplete imaging views, and certain imaging artifacts such as excessive motion blurring and certain types of incidental findings. Hence, in a further aspect, the images extracted from the screen scrape of the controller display can be sent to the radiologist for use in the review. These are at lower resolution, and can be sent via an electronic data transfer connection other than via the PACS, thus speeding delivery of reviewable images.

In yet a further aspect, a Radiology Information System (RIS) or other available databases can be automatically mined to provide ancillary information for assisting the radiologist. For example, if this is a follow-up imaging examination then the images of a prior examination can be brought up and shown side-by-side, and/or the radiology report summarizing the prior examination can be retrieved and provided to the radiologist reviewing the current examination images.

In some embodiments disclosed herein, the images and subsequent radiologist corrections (e.g., additional image views ordered by the radiologist, corrected field-of-view, incidental findings noticed by the reviewing radiologist, or so forth) can be collected to form a database that can be used to train machine learning (ML) components to automatically detect some problems, or incidental findings. This enables providing automated suggestions to the technician or alerting the radiologist to a possible incidental finding, thus further increasing the efficiency and effectiveness of the radiologist review.

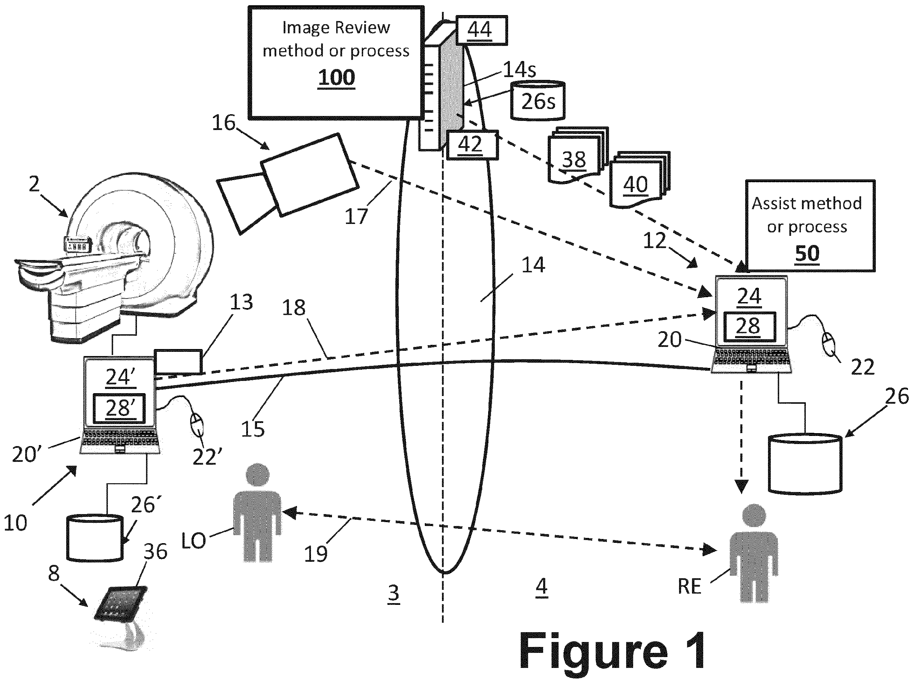

With reference to Fig. 1, an apparatus for providing assistance from a remote medical imaging expert RE (e.g., a remote imaging expert) to a local imaging technician or local technician operator LO is shown. Such a system is also referred to herein as a radiology operations command center (ROCC). As shown in Fig. 1, the local operator LO, who operates a medical imaging device (also referred to as an image acquisition device, imaging device, and so forth) 2, is located in a medical imaging device bay 3, and the remote expert RE is disposed in a remote location 4. It should be noted that the remote expert RE may not necessarily directly operate the medical imaging device 2, but rather provides assistance to the local operator LO in the form of advice, guidance, instructions, or the like. Moreover, as further disclosed herein, for the purposes of providing notification to an on-call radiologist to review the images before releasing the patient, the remote expert RE may be the on-call radiologist. As a further point of clarification, the location 4 of the remote expert RE (e.g., the on-call radiologist) may be in the same building or even on the same floor or radiology department as the local technician operator LO - the term "remote" means the remote expert RE is not in the same imaging bay or in the adjoining control room (if any) from which the local operator LO conducts the medical imaging examination.

The image acquisition device 2 can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device 2 may also be a hybrid imaging device such as a PET/CT or SPECT/CT imaging system. While a single image acquisition device 2 is shown by way of illustration in Fig. 1, more typically a medical imaging laboratory will have multiple image acquisition devices, which may be of the same and/or different imaging modalities. For example, if a hospital performs many CT imaging examinations and relatively fewer MRI examinations and still fewer PET examinations, then the hospital's imaging laboratory (sometimes called the "radiology lab" or some other similar nomenclature) may have three CT scanners, two MRI scanners, and only a single PET scanner. This is merely an example. Moreover, in an ROCC setting the remote location 4 may provide service to multiple hospitals, and/or there may be multiple remote experts RE. The local operator LO controls the medical imaging device 2 via an imaging device controller 10. The remote expert RE is stationed at a remote workstation 12 (or, more generally, at an electronic processing device 12).

The imaging device controller 10 includes an electronic processor 20', at least one user input device such as a mouse 22', a keyboard, and/or so forth, and a display device 24'. The imaging device controller 10 presents a device controller graphical user interface (GUI) 28' on the display 24' of the imaging device controller 10, via which the local operator LO accesses device controller GUI screens for entering the imaging examination information such as the name of the local operator LO, the name of the patient and other relevant patient information (e.g. gender, age, etc.) and for controlling the (typically robotic) patient support to load the patient into the bore or imaging examination region of the imaging device 2, selecting and configuring the imaging sequence(s) to be performed, acquiring preview scans to verify positioning of the patient, executing the selected and configured imaging sequences to acquire clinical images, display the acquired clinical images for review, and ultimately store the final clinical images to a Picture Archiving and Communication System (PACS) or other imaging examinations database.

As diagrammatically shown in Fig. 1, in some embodiments, a camera 16 (e.g., a video camera) is optionally arranged to acquire a video stream 17 of a portion of the medical imaging device bay 3 that includes at least the area of the imaging device 2 where the local operator LO interacts with the patient, and optionally may further include the imaging device controller 10 (or optionally another device such as a contrast injector controller (not shown)). The video stream 17 is sent to the remote workstation 12 via the communication link 14, e.g., as a streaming video feed received via a secure Internet link.

In other embodiments, the live video feed 17 of the display 24' of the imaging device controller 10 is, in the illustrative embodiment, provided by a video cable splitter 15 (e.g., a DVI splitter, a HDMI splitter, and so forth). In other embodiments, the live video feed 17 may be provided by a video cable connecting an auxiliary video output (e.g. aux vid out) port of the imaging device controller 10 to the remote workstation 12 of the operated by the remote expert RE. Alternatively, a screen mirroring data stream 18 is generated by screen sharing software 13 running on the imaging device controller 10 which captures a real-time copy of the display 24' of the imaging device controller 10, and this copy is sent from the imaging device controller 10 to the remote workstation 12. These are merely nonlimiting illustrative examples.

The communication link 14 also provides a natural language communication pathway 19 for verbal and/or textual communication between the local operator LO and the remote expert RE, in order to enable the latter to assist the former in performing the imaging examination. For example, the natural language communication link 19 may be a Voice-Over-Internet-Protocol (VOIP) telephonic connection, a videoconferencing service, an online video chat link, a computerized instant messaging service, or so forth. Alternatively, the natural language communication pathway 19 may be provided by a dedicated communication link that is separate from the communication link 14 providing the data communications 17, 18, e.g., the natural language communication pathway 19 may be provided via a landline telephone. These are again merely nonlimiting illustrative examples.

Fig. 1 also shows, in the location 4 of the remote expert RE including the remote workstation 12, such as an electronic processing device, a workstation computer, or more generally a computer, which is operatively connected to receive and present the video 17 of the medical imaging device bay 3 from the camera 16 and to present the screen mirroring data stream 18 as a mirrored screen. Additionally or alternatively, the remote workstation 12 can be embodied as a server computer or a plurality of server computers, e.g., interconnected to form a server cluster, cloud computing resource, or so forth. The workstation 12 includes typical components, such as an electronic processor 20 (e.g., a microprocessor), at least one user input device (e.g., a mouse, a keyboard, a trackball, and/or the like) 22, and at least one display device 24 (e.g., an LCD display, plasma display, cathode ray tube display, and/or so forth). In some embodiments, the display device 24 can be a separate component from the workstation 12. The electronic processor 20 is operatively connected with a one or more non-transitory storage media 26. The non-transitory storage media 26 may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation 12, various combinations thereof, or so forth. It is to be understood that any reference to a non-transitory medium or media 26 herein is to be broadly construed as encompassing a single medium or multiple media of the same or different types. Likewise, the electronic processor 20 may be embodied as a single electronic processor or as two or more electronic processors. The non-transitory storage media 26 stores instructions executable by the at least one electronic processor 20. The instructions include instructions to generate a graphical user interface (GUI) 28 for display on the remote expert display device 24.

The medical imaging device controller 10 in the medical imaging device bay 3 also includes similar components as the remote workstation 12 disposed in the remote service center 4. Except as otherwise indicated herein, features of the medical imaging device controller 10 disposed in the medical imaging device bay 3 similar to those of the remote workstation 12 disposed in the remote service center 4 have a common reference number followed by a "prime" symbol (e.g., processor 20', display 24', GUI 28') as already described. In particular, the medical imaging device controller 10 is configured to display the imaging device controller GUI 28' on a display device or controller display 24' that presents information pertaining to the control of the medical imaging device 2 as already described, such as imaging acquisition monitoring information, presentation of acquired medical images, and so forth. The real-time copy of the display 24' of the controller 10 provided by the video cable splitter 15 or the screen mirroring data stream 18 carries the content presented on the display device 24' of the medical imaging device controller 10. The communication link 14 allows for screen sharing from the display device 24' in the medical imaging device bay 3 to the display device 24 in the remote service center 4. The GUI 28' includes one or more dialog screens, including, for example, an examination/scan selection dialog screen, a scan settings dialog screen, an acquisition monitoring dialog screen, among others. The GUI 28' can be included in the video feed 17 or provided by the video cable splitter 15 or by the mirroring data stream 17' and displayed on the remote workstation display 24 at the remote location 4.

Fig. 1 shows an illustrative local operator LO, and an illustrative remote expert RE. In an ROCC, a staff of remote experts may be provided who are expert imaging technicians or operators who are made available by the ROCC to assist local operators LO at different hospitals, radiology labs, or the like. The ROCC may be housed in a single physical location or may be geographically distributed. The server computer 14s is operatively connected with a one or more non-transitory storage media 26s. The non-transitory storage media 26s may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the server computer 14s, various combinations thereof, or so forth. It is to be understood that any reference to a non-transitory medium or media 26s herein is to be broadly construed as encompassing a single medium or multiple media of the same or different types. Likewise, the server computer 14s may be embodied as a single electronic processor or as two or more electronic processors. The non-transitory storage media 26s stores instructions executable by the server computer 14s. In addition, the non-transitory computer readable medium 26s (or another database) stores data related to a set of remote experts RE and/or a set of local operators LO. The remote expert data can include, for example, skill set data, work experience data, data related to ability to work on multi-vendor modalities, data related to experience with the local operator LO and so forth.

With continuing reference to Fig. 1, when the local operator LO requires assistance from the remote expert RE of the ROCC in performing an imaging examination, the communication link 14 connects the local operator LO/ remote expert RE. The GUI 28 is provided as a remote assistance UI on the display device 24 operable by a remote expert RE. The UI 28 provides two-way communication between the local operator LO and the remote expert RE via which the remote expert can provide assistance to the local medical imaging device operator LO.

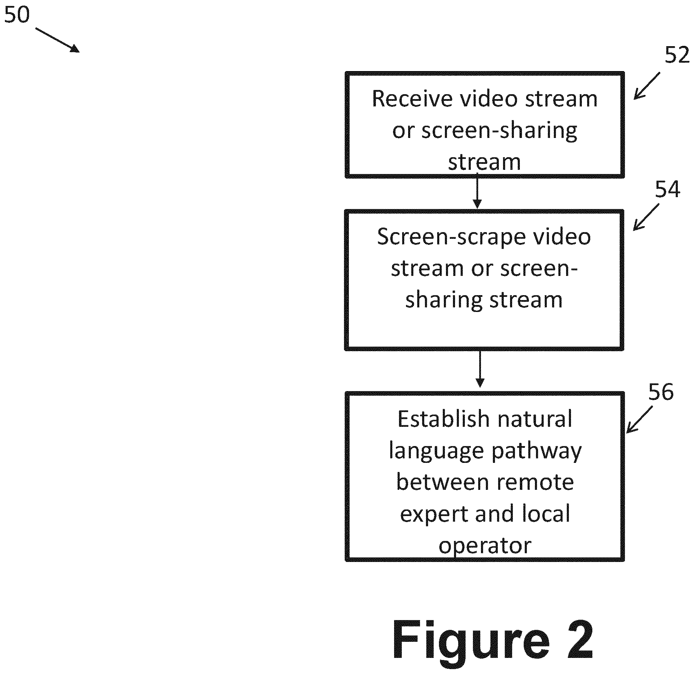

With continuing reference to Fig. 1 and with further reference now to Fig. 2, The remote workstation 12 of the selected remote expert RE, and/or the medical imaging device controller 10 being run by the local operator LO, is configured to perform a method or process 50 for providing assistance from the remote expert RE to the local operator LO. For brevity, the method 50 will be described as being performed by the remote workstation 12. The non-transitory storage medium 26 stores instructions which are readable and executable by the at least one electronic processor 20 (of the workstation 12, as shown, and/or the electronic processor or processors of a server or servers on a local area network or the Internet) to perform disclosed operations including performing the method or process 50.

A suitable implementation of the assistance method or process 50 is as follows. The method 50 is performed over the course of (at least a portion of) an imaging procedure performed using the medical imaging device 2, and the remote expert RE is in this method or process 50 an expert imaging technician. To perform the method 50, at an operation 52, the workstation 12 in the remote location 4 is programmed to receive at least one of: (i) the video 17 from the video camera 16 of the medical imaging device 2 located in the medical imaging device bay 3; and/or (ii) the screen sharing 18 from the screen sharing software 13; and/or (iii) the video 17 tapped by the video cable splitter 15. The video feed 17 and/or the screen sharing 18 can be displayed at the remote workstation display 24, typically in separate windows of the GUI 28. At an operation 54, the video feed 17 and/or the screen sharing 18 can be screen-scraped to determine information related to the medical imaging examination (e.g., modality, vendor, anatomy to be imaged, cause of issue to be resolved, and so forth). In particular, the GUI 28 presented on the display 24 of the remote workstation 12 preferably includes a window presenting the video 17, and a window presenting the mirrored screen of the medical imaging device controller 10 constructed from the screen mirroring data stream 18, and status information on the medical imaging examination that is maintained at least in part using the screen-scraped information. This allows the remote expert RE to be aware of the content of the display of the medical imaging device controller 10 (via the shared screen) and also to be aware of the physical situation, e.g., position of the patient in the medical imaging device 2 (via the video 17), and to additionally be aware of the status of the imaging examination as summarized by the status information. During an imaging procedure, at an operation 56, the natural language communication pathway 19 is established between the remote medical professional workstation 12 and the ROCC device 8, and is suitably used to allow the local operator LO and the remote expert RE to discuss the procedure and in particular to allow the remote expert to provide advice to the local operator LO.

The ROCC framework such as that described above with reference to Figs. 1 and 2 provides the local operator LO with access during an imaging examination to the assistance of a remote expert RE who is an expert imaging technician or operator. This remote expert can provide assistance in performing the imaging examination, such as guidance on how to position the patient, guidance on setting up imaging scan configurations, guidance on operation of the imaging device 2 or ancillary equipment (e.g., a contrast agent injector or so forth), and so forth.

It is further disclosed herein to leverage the ROCC to provide expedited review of an imaging examination by an on-call radiologist prior to releasing the patient. For this use case of the ROCC, the remote expert RE is suitably an on-call radiologist. That on-call radiologist is a trained medical doctor (e.g., typically an M.D. in the United States) who has a wide range of duties only one of which is providing on-call review of images of a completed imaging examination prior to releasing the patient. Conventionally, the radiologist would be called by the local operator LO to review the images at the end of the imaging examination. At that point, the radiologist would download the images from the PACS in order to review them. This conventional approach has substantial disadvantage. The radiologist is not notified of the availability of the images for review until the local operator LO has time to call the radiologist, which will typically be after the imaging acquisition is fully completed and may also be after the local operator LO has uploaded the clinical images to the PACS. Furthermore, those clinical images are of high resolution and hence are large digital files, so that the download of the images to the radiologist's work station can take additional time. Still further, the radiologist has no immediate information about the examination other than whatever the radiologist can glean from the examination order, any information provided by the local operator LO, and the images themselves. Hence, for example, the radiologist may be unaware of a relevant prior imaging examination of the same patient.

In embodiments disclosed herein, the ROCC is leveraged to provide more timely notification to the radiologist of availability of images for review. In this use case of the ROCC, the remote expert RE is the on-call radiologist, and the ROCC automatically notifies the remote expert/on-call radiologist RE when the imaging examination is finished or, in some embodiments, a short time before the examination is finished. Furthermore, in some embodiments, the scraped controller screen is mined to provide the images for review by the on-call radiologist. This approach leverages the fact that the clinical images are displayed on the controller display for review by the local operator LO, albeit at significantly lower resolution (i.e., "screen" resolution) compared with the full-resolution clinical images. Although at lower resolution, these "screen" images obtained from the scraped controller display are sufficient for the on-call radiologist to identify issues such as incorrect field-of-view, missing views, certain imaging artifacts such as excessive motion blurring and certain types of incidental findings. Still further, in some embodiments, the ROCC automatically checks available databases for other information that may be relevant to the radiologist review, such as prior radiology reports on the same patient, and provides those to the radiologist for consideration.

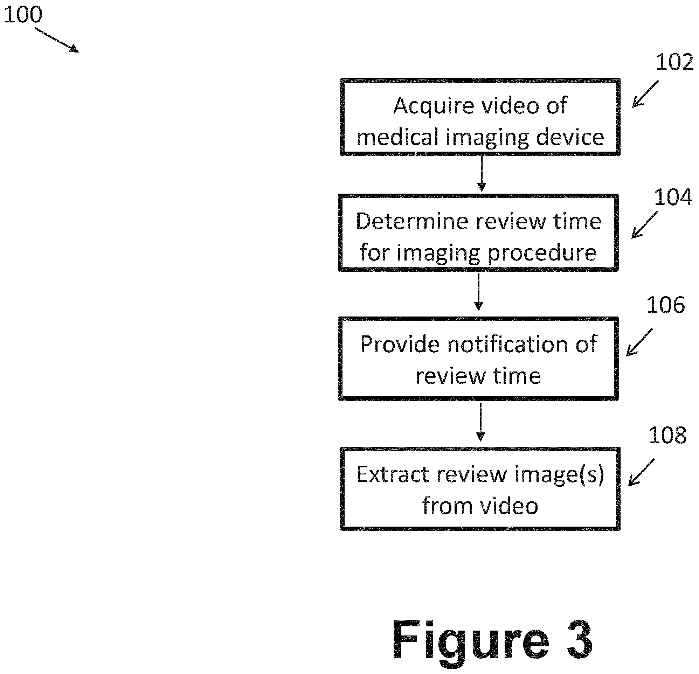

With reference to Fig. 3, and with continuing reference to Fig. 1, an illustrative embodiment of a method 100 of the use case in which the ROCC provides notification to an on-call radiologist of images ready for review is diagrammatically shown as a flowchart. In the method 100, the remote expert RE is the on-call radiologist. At an operation 102, the video 17 of the medical imaging device 2 is acquired. In some embodiments, the acquiring operation 102 includes acquiring the video from the camera 16 disposed in the medical imaging bay 3. In some embodiments, the acquiring operation 102 includes screen-scraping image frames of the medical imaging device 2.

At an operation 104, a review time for the medical imaging procedure is determined. Determining the review time is preferably based on obtaining information from the medical procedure that indicates a fixed time point in the medical procedure, for instance a start of the procedure or of a specific step in the procedure an end of the procedure or of a specific step in the procedure. In a particularly preferably embodiment the determination operation 104 is based on detecting the fixed time point from the acquired video 17. In one embodiment, the determination operation 104 includes detecting administration of a contrast agent to a patient for which the medical imaging procedure is being performed. In another embodiment, the determination operation 104 includes detecting, from the acquired video 17, text indicating a time point of the medical imaging procedure, and determining the review time from a time of the detection of the time point. The "time point" as used herein refers to any identifiable time in medical imaging procedure exam from which the review time can be estimated. For example, it might detect start of the last imaging sequence, which is known to take 3 minutes, so then the review time is that time point plus 3 minutes.

At an operation 106, a notification of the determined review time is provided to the remote workstation 12. At an operation 108, at the review time, at least one review image 38 is extracted from the video 17, and made available at the remote workstation 12. This can be done, for example, based on a template of the controller display identifying where the "screen" image of the clinical images are shown. While operation 108 is shown in Fig. 3 as following notification operation 106, more generally the operation 108 may identify and store relevant images for review over the course of the imaging examination. For example, if the imaging examination acquires multiple views of the target anatomy (for example, axial, sagittal, and coronal views), these may be acquired in separate image scans performed sequentially, and the ROCC performs the operation 108 at the end of each such image scan to acquire the respective axial, sagittal, and coronal view images. In some embodiments, the review image(s) 38 are sent to the remote workstation 12. In other embodiments, a link connected to the review image(s) 38 is sent to the remote workstation 12.

In one example, the review image(s) 38 are transmitted to the remote workstation 12 without first storing the review image(s) 38 on a Picture Archiving and Communication System (PACS). Indeed, if the images are scraped "screen" images, they may never be stored in the PACS. (Rather, only the full-resolution clinical images are stored in the PACS). In another example, the review image(s) 38 are transmitted to the remote workstation 12, and displayed on the display device 24 using a hanging protocol for the medical imaging procedure. In a further example, the review image(s) 38 are transmitted to the remote workstation 12, and the medical imaging procedure acquires at least one clinical image 40 corresponding to the at least one review image 38, and the at least one review image 38 is at a lower resolution than the corresponding at least one clinical image 40.

In some embodiments, errors impacting a quality of the at least one review image 38 is detected by automated analysis of the at least one review image 38. In other embodiments, one or more user inputs indicative of a correction to the at least one review image 38 can be input by the remote expert RE via the remote workstation 12. The review image(s) 38 can then be updated with one or more annotations 42 based on the user inputs. In some embodiments, a machine-learning (ML) component 44 (e.g., an artificial neural network (ANN)) implemented in the server computer 14s can be trained with the review image(s) and the annotation(s) 42. The ML component 44 can be used to automatically detect findings in the clinical images 40 and/or detect an issue in the at least one review image 38.

In some embodiments, the reviews image(s) 38 and/or the clinical image(s) 40 can presented locally and/or transmitted to the remote electronic processing device 12 prior to the completion of the medical imaging examination. While the clinical image(s) 40 can be partially reconstructed and may be inadequate for diagnostic use, they should be acceptable for imaging protocol and set-up quality assessment. As such, based on the imaging protocol or based on the image reconstruction status, a ready for review time can be determined. This can beneficially expedite completion of the examination and unloading of the patient, thus improving workflow efficiency.

Regardless of whether it is just-in-time presentation or based on incomplete reconstruction, the remote expert RE advantageously receives notice (e.g., a forecast or countdown) of when the clinical image(s) 40 for quality check will be available, thus facilitating prompt feedback by the remote expert RE to expedite workflow efficiency. By knowing the approximate (or in some cases exact) time the images will be available, the remote expert RE can adjust his or her schedule to accommodate the image review, or can merely be prepared to review as soon as the clinical image(s) 40 are available, thereby ensuring that the time to relay the clinical image(s) 40 to the remote expert RE and obtain a response does not introduce additional imaging procedure time (e.g. time patient has to be in the imaging system, such as an MR).

In some embodiments, the estimated time until the clinical image(s) 40 are available not only accounts for when the clinical image(s) 40 are estimated to be ready, but when they would be ready for review remotely, thus including any transmission time.

If the current examination is a follow-up imaging examination for which there are potentially useful prior images from a prior imaging study, then the prior review image(s) 38 data can be sent ahead of current review image(s) 38, thereby ensuring it is available when the read is to be done. In some embodiments, when the prior information is made available it can indicate whether prior review by the remote expert RE would be beneficial (time-saving) as opposed to merely looking at a side-by-side (which would not allow for time-savings). Such time for review of priors can be subtracted from the time until review to ensure the remote expert RE is ready to review the current images once available.

### EXAMPLE

The discloses ROCC system is a highly secure collaboration platform that enables virtualized imaging operations. Virtual scanner access (i.e., the ability for expert users located remotely to view and access scanner console screens from afar) is a fundamental ROCC enabler. ROCC provides access to the imaging console screens; in turn, the information retrieved from the console screens could be used to make meaningful changes in the radiology workflows ensuring better exam quality and reduced number of call-backs. If done haphazardly, mid-exam image review by radiologists may extend procedure durations, tie up scanners, fatigue patients, stress the staff, etc. Therefore, radiologists have to be able to perform these real-time image reviews in a timely manner.

The disclosed ROCC system can use information scraped from imaging console screens to predict when examination is close to completion and send alerts to on-call radiologists regarding forthcoming image review. For example, for MR imaging systems 2, a summation of prescribed sequences can be used as a proxy for time remaining in the scan, while with CT imaging systems 2, contrast injections could trigger "End of Exam" alerts, etc. Recipients for the alerts could be pre-set based on established schedule or selected by a local technologist or a remote expert based on *a priori* knowledge at the beginning of the scan. Potentially, some exams would benefit from mid-exam radiologist review more than others; therefore, the system may generate alerts based on a set of conditions.

Timeliness is extremely important. However, if a radiologist was to rely on existing infrastructure (i.e. wait for images to be sent to PACS, identify the study, load the correct images (which can be time consuming especially when done remotely), etc. the process would take prohibitively long. Images can be scraped from console screens to create a summary report for radiologists to review. Protocol-specific summary reports would contain all the prerequisite views. An algorithm would match scraped images against the protocol-based template and populate the report as exam progresses. A radiologist may feasibly review the images even before the exam is finished. Reports could be customized based on protocol, reviewing radiologist, even patient if necessary.

Providing highly abstracted, scraped images for radiologists to review without supplying some context may result in missed opportunities. For instance, if a patient's prostate cancer has been followed by annual MR exams over the course of 3 years and the current exam is part of a systematic follow-up, it is important to review prior imaging to ensure consistency of follow-up. This could be done by a radiologist if appropriate priors are presented as part of the end of exam review dossier but better yet - automatically. One of the ways in which the need for additional imaging can be evaluated is to ensure consistency of acquired imaging in follow-up cases. This could be done by ensuring consistency in 1) chosen protocols, 2) acquired imaging sequences, 3) scan parameters (field of view, slice thickness, injection delays, etc.). By flagging incomplete follow-up exams or providing certain prior exam views for radiologists would allow for a more meaningful review process.

As radiologists start reviewing exams and requesting additional scanning - each exam becomes a learning opportunity. The disclosed ROCC system can further build upon mid-scan radiologist review by 1) collecting data detailing types of protocols used, types of repeat imaging requested, technologists and radiologists involved, outcomes, key performance indicators (KPIs), etc. and 2) using this curated data build AI models (i.e. based on reinforcement learning). Artificial intelligence (AI) models could proactively flag exams likely to require additional imaging and either provide direct feedback to the technologist, alert the expert user, or make sure such cases get routed for mid-scan review by a radiologist prior to patient's departure.

As radiologists start reviewing exams and requesting additional imaging - some of the additional imaging will be done to better assess incidental findings. This will provide the ROCC system with a unique opportunity to build a repository of images and use this data to train algorithms for incidental findings detection. the ROCC system will query reports, transcripts of rad-tech interactions, survey radiologists, etc. in order to accurately curate the nature of additional imaging done at the time of the exam. In one iteration, as exam progresses and the images are being acquired the algorithm is continuously running, analyzing the streaming videos. If any of the images are flagged for suspicious findings, the output from the model is included in the report and reviewed by a radiologist prior to patient's release.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

In the foregoing detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials, and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

## Claims

1. A non-transitory computer readable medium (26s) storing instructions executable by at least one electronic processor (14s) to perform a method (100) to coordinate radiologist review of a medical imaging procedure performed using a medical imaging device (2), the method comprising:
acquiring a video (17) of the medical imaging device;
determining a review time for the medical imaging procedure;
providing a notification to a remote electronic processing device (12) operable by a radiologist of the determined review time; and
at the review time, extracting at least one review image (38) from the video and making the at least one review image available at the remote electronic processing device.

2. The non-transitory computer readable medium (26s) of claim 1, wherein the review time is determined based on obtaining information from the medical procedure, preferably from the acquired video, that indicates a fixed time point in the medical imaging procedure, for instance including one or more of a start of the procedure or of a specific step in the procedure an end of the procedure or of a specific step in the procedure.

3. The non-transitory computer readable medium (26s) of claim 1 or 2, wherein determining a review time includes:
detecting administration of a contrast agent to a patient for which the medical imaging procedure is being performed.

4. The non-transitory computer readable medium (26s) of claim 1 or 2, wherein determining a review time includes:
detecting, from the acquired video (17), text indicating a time point of the medical imaging procedure; and
determining the review time from a time of the detection of the time point.

5. The non-transitory computer readable medium (26s) of any one of claims 1-4, wherein the acquiring a video (17) of the medical imaging device (2) includes:
acquiring the video from a camera (16) disposed in a medical imaging bay (3) in which the medical imaging device is disposed.

6. The non-transitory computer readable medium (26s) of any one of claims 1-4, wherein the acquiring a video (17) of the medical imaging device (2) includes:
screen-scraping image frames of the medical imaging device.

7. The non-transitory computer readable medium (26s) of any one of claims 1-4, wherein the method (100) further includes:
transmitting the at least one review image to the remote electronic processing device (12) without first storing the at least one review image on a Picture Archiving and Communication System (PACS).

8. The non-transitory computer readable medium (26s) of any one of claims 1-7, wherein the at least one review image (38) comprises a plurality of review images and the method (100) further includes:
transmitting the review images to the remote electronic processing device (12); and
displaying the review images using a hanging protocol for the medical imaging procedure.

9. The non-transitory computer readable medium (26s) of any one of claims 1-8, wherein the method (100) further includes:
transmitting the at least one review image (38) to the remote electronic processing device (12);
wherein the medical imaging procedure acquires at least one clinical image (40) corresponding to the at least one review image, and the at least one review image is at a lower resolution than the corresponding at least one clinical image.

10. The non-transitory computer readable medium (26s) of any one of claims 1-9, wherein the method (100) further includes:
detecting errors impacting a quality of the at least one review image (38) by automated analysis of the at least one review image.

11. The non-transitory computer readable medium (26s) of claim 10, wherein the method (100) further includes:
receiving user inputs from the radiologist indicative of a correction to the at least one review image (38); and
updating the at least one review image with one or more annotations (42) based on the user inputs.

12. The non-transitory computer readable medium (26s) of claim 11, wherein the method (100) further includes:
training a machine-learning (ML) component (44) with the at least one review image (38) and the one or more annotations (40).

13. The non-transitory computer readable medium (26s) of claim 12, wherein the method (100) further includes:
using the trained ML component (44) to automatically detect findings in at least one clinical image (40) and/or detect an issue in the at least one review image (38).

14. The non-transitory computer readable medium (26s) of any one of claims 1-13, wherein extracting at least one review image (38) from the video and making the at least one review image available at the remote electronic processing device (12) includes:
transmitting the at least one review image or at least one clinical image (40) to the remote electronic processing device prior to completion of a medical imaging procedure; and
determining a review time for the remote expert (RE) to review the clinical image.

15. A method (100) to coordinate review of a medical procedure performed using a medical device (2), the method comprising:
acquiring a video (17) of the medical device;
determining a review time for the medical procedure;
providing a notification to a remote electronic processing device (12) operable by a medical professional of the determined review time; and
at the review time, extracting at least one review image (38) from the video and making the at least one review image available at the remote electronic processing device.
